Europäisches Patentamt

European Patent Office

Office européen des brevets

(11) Veröffentlichungsnummer: **0 154 830**
**B1**

(12) # EUROPÄISCHE PATENTSCHRIFT

(45) Veröffentlichungstag der Patentschrift:
06.05.87

(21) Anmeldenummer: 85101611.3

(22) Anmeldetag: 14.02.85

(51) Int. Cl.⁴: **C 07 C 39/07**, C 07 C 37/16,
C 07 C 37/48

(54) **Verfahren zur Umwandlung von meta/para-Kresolgemischen.**

(30) Priorität: 23.02.84 DE 3406536

(43) Veröffentlichungstag der Anmeldung:
18.09.85 Patentblatt 85/38

(45) Bekanntmachung des Hinweises auf die Patenterteilung: 06.05.87 Patentblatt 87/19

(84) Benannte Vertragsstaaten:
BE CH DE FR GB IT LI NL

(56) Entgegenhaltungen:
EP-A- 0 032 974
EP-A- 0 119 421
DE-A- 1 804 632
FR-A- 2 103 244
GB-A- 2 072 674

(73) Patentinhaber: **Union Rheinische Braunkohlen
Kraftstoff Aktiengesellschaft
Ludwigshafener Strasse o. Nr. Postfach 8
D-5047 Wesseling (DE)**

(72) Erfinder: **Korff, Joachim, Dr.
Kapellenstrasse 28
D-5303 Bornheim-Merten (DE)**
Erfinder: **Keim, Karl-Heinz
Höhenring 31
D-5351 Heimerzheim (DE)**

## Beschreibung

Die Erfindung betrifft eine Verfahren zur Umwandlung von meta-/para-Kresolgemischen durch katalytische Methylierung in den ortho-Positionen, Trennung der gebildeten 2,3,6- und 2,4,6-Trimethylphenole und anschließende Umwandlung des 2,4,6-Trimethylphenols in Gegenwart eines Katalysators, der wenigstens 60 Gew.% Eisenoxid(e) enthält, in ein Gemisch von Methylphenolen, das im wesentlichen frei von meta-substituierten Methylphenolen ist.

Die katalytische Methylierung von meta-Kresol zu 2,3,6-Trimethylphenol ist bekannt. So erhält man gemäß DE-PS 30 12 357 (s. auch Nachanmeldung GB-A-20 72 674) mit Eisenoxid enthaltenden Katalysatoren in 99,5 %iger Selektivität 2,3,6-Trimethylphenol bei Einsatz von reinem m-Kresol. Setzt man gemäß diesem Stand der Technik reines p-Kresol mit Methanol oder Dimethylether mit den genannten Katalysatoren um, so erhält man 2,4,6-Trimethylphenol in 99 %iger Selektivität. Auch in EP-A1-32 974 ist die Herstellung von 2,3,6-Trimethylphenol durch Methylierung von m-Kresol beschrieben.

Es sind zahlreiche andere Katalysatoren bekannt, die ebenfalls Phenole in hoher Selektivität in o-Stellung substituieren. So wird in DE-OS 27 16 035 ein Kupfer-Chromoxid-Katalysator beschrieben, der zu selektiver o-Substitution führt. Gemäß DE-AS 25 47 309 eignen sich $Fe_2O_3/SiO_2/Cr_2O_3$ bzw. $Fe_2O_3/SiO_3/Cr_2O_3/K_2O$-Katalysatoren zur o-Methylierung von Phenolen.

2,3,6-Trimethylphenol ist ein wichtiges Ausgangsmaterial zur Herstellung von 2,3,6-Trimethylhydrochinon, das als Baustein zur Vitamin E-Synthese benötigt wird.

Eines der technisch angewandten Verfahren zur Herstellung von 2,3,6-Trimethylphenol geht aus von offenkettigen Bausteinen, die zu einem Sechsring kondensiert werden, wonach anschließend durch Dehydrierung Aromatisierung zum Phenolring herbeigeführt wird. Dieses Verfahren ist u. a. in DE-AS 16 68 874 und DE-PS 17 93 037 beschrieben.

Methyliert man gemäß dem Stand der Technik relativ leicht zugängliche und preiswerte Gemische von meta- und para-Kresol, so erhält man jedoch neben dem erwünschten 2,3,6-Trimethylphenol je nach p-Kresolanteil, das unerwünschte, nach dem Stand der Technik nicht verwertbare 2,4,6-Trimethylphenol. Selektive Isomerisierungen von 2,4,6-Trimethylphenol in Gegenwart von Phenol und/oder Phenolderivaten sind in P 33 04 663.8-42 von der gleichen Anmelderin beschrieben worden, wobei ein Produktgemisch erhalten wird daß im wesentlichen frei ist von m-substituierten Alkylphenolen.

Ein Verfahren zur katalytischen Transmethylierung methyl-substituierter Phenole ist auch in DE-A-18 04 632 beschrieben. Gemäß dieser Anmeldung werden jedoch auch m-Isomere in erheblichen Mengen gebildet.

Gemäß der erweiterten Europäischen Anmeldung EP-A-01 19 421 unter Inanspruchnahme der Priorität der deutschen Patensanmeldung 33 04 663.8-42 hat die Anmelderin gefunden, daß sich Eisenoxid(e) allein sowie zahlreiche Kombinationen von Eisenoxid(en) mit anderen Oxiden hervorragend für die selektive Isomerisierung und Transalkylierung von 2,4,6-Trimethylphenol eignen.

Auch Verfahren zur Trennung von 2,3,6-Trimethylphenol und 2,4,6-Trimethylphenol sind bekannt. Ein Verfahren ist beispielsweise in FR-A-21 03 244 offenbart.

Die bisher ungelöste Aufgabe, 2,3,6-Trimethylphenol aus m-/p-Kresolgemischen herzustellen, ohne ein unerwünschtes Nebenprodukt in Kauf nehmen zu müssen, wurde erfindungsgemäß in bisher unerreichter Weise durch die Kombination der selektiven o-Methylierung von meta-/para-Kresolen und selektiven Isomerisierung und Transalkylierung des 2,4,6-Trimethylphenols gelöst.

Die Erfindung betrifft daher ein Verfahren zur Umwandlung von meta-/para-Kresolgemischen und meta-/para-Kresolgemische enthaltenden Gemischen, dadurch gekennzeichnet, daß das meta-/para-Kresolgemisch durch katalytische Umsetzung mit Methanol und/oder Dimethylether bei 270-450 °C bei einer Verweilzeit von 0,01-10 Sekunden und einem molaren Methylphenol zu Methanol- und/oder Dimethyletherverhältnis von 1 : 0,1-10 in ein Gemisch von 2,3,6-Trimethylphenol und 2,4,6-Trimethylphenol umgewandelt bzw. im wesentlichen umgewandelt wird, das 2,3,6-Trimethylphenol vom 2,4,6-Trimethylphenol abgetrennt bzw. im wesentlichen abgetrennt wird und das 2,4,6-Trimethylphenol oder ein im wesentlichen 2,4,6-Trimethylphenol enthaltendes Gemisch in Gegenwart von Phenol und/oder alkylierten Phenolen, in Gegenwart eines Katalysators, der aus Eisenoxid(en) besteht oder mindestens 60 Gew.% Eisenoxid(e) und wenigstens ein Oxid aus wenigstens einer der Gruppen

1. B, Al, Ce, Ga, In, Sc, Y
2. Si, Ge, Sn, Pb, Ti, Zr, Hf
3. Cr, V, Nb, Ta, Mo, W, Re, Co, Ni, Ru, Ir
4. Li, Na, K, Rb, Cs, Be, Mg, Ca, Sr, Ba, Mn, La, Cu, Zn, Cd

enthält, bei Temperaturen von 200-550 °C, Drücken von 1-300 bas (bei Reaktionstemperatur) und Verweilzeiten von 0,1-10 h in Inertgasatmosphäre in ein Gemisch von methylierten Phenolen umgewandelt wird, das im wesentlichen frei von meta-substituierten Methylphenolen ist.

Als Produkte entstehen erfindungsgemäß 2,3,6-Trimethylphenol, und aus 2,4,6-Trimethylphenol o-Kresol, p-Kresol, 2,4-Dimethylphenol und 2,6-Dimethylphenol. m-Alkylphenole wie m-Kresol, 2,5-Dimethylphenol und 2,3-Dimethylphenol werden praktisch nicht gebildet.

m-Alkylphenole sind selbstverständlich dann im Produkt enthalten, wenn keine vollständige Abtrennung des 2,3,6-Trimethylphenols vor der Isomerisierungs-/Transalkylierungsstufe durchgeführt worden ist, bzw. wenn 2,4,6-Trimethylphenol in die Isomerisierungs-/Transalkylierungsstufe eingesetzt wird, das m-substituierte Methylphenole als Beimengungen enthält.

Da die aus 2,4,6-Trimethyphenol durch Isomerisierung und Transalkylierung in Gegenwart von Phenol erfindungsgemäß erhaltenen Produkte ausschließlich wertvolle Alkylphenole für zahlreiche Verwendungen sind, ist es mit der erfindungsgemäßen Kombination von Reaktionsschritten erstmals gelungen, 2,3,6-Trimethylphenol in einer bisher unerreicht wirtschaftlichen Weise aus einem leicht zugänglichen Einsatzprodukt herzustellen.

Die o-Substitution von Phenolen, die wenigstens eine freie o-Stellung besitzen, durch Methylgruppen in Gegenwart oxidischer Katalysatoren ist, wie oben ausgeführt, an sich bekannt.

Ebenso ist die Trennung von 2,3,6-Trimethylphenol und 2,4,6-Trimethylphenol bekannt. Sie erfolgt üblicherweise durch fraktionierte Destillation. Diese kann mit oder Anwendung von Vakuum erfolgen, jedoch auch andere Trennverfahren wie z. B. fraktionierte Kristallisation oder Trennung über Molsiebe sind anwendbar.

Auch die Isomerisierung von 2,4,6-Trimethylphenol mit Eisenoxid(e) enthaltenden Katalysatoren ist in der nicht vorveröffentlichten EP-A-01 19 421 von der gleichen Anmelderin beschrieben worden, wobei die Katalysatoren zur Isomerisierung und Transalkylierung des 2,4,6-Trimethylphenols
— aus Eisenoxid(en) bestehen oder
— mindestens 60 Gew.% Eisenoxid(e) und wenigstens ein Oxid aus wenigstens einer der folgenden Gruppen enthalten :

1. B, Al, Ce, Ga, In, Sc, Y
2. Si, Ge, Sn, Pb, Ti, Zr, Hf
3. Cr, V, Nb, Ta, Mo, W, Re, Co, Ni, Ru, Ir
4. Li, Na, K, Rb, Cs, Be, Mg, Ca, Sr, Ba, Mn, La, Cu, Zn, Cd.

Die Anwendung der erfindungsgemäßen Kombination dieser Verfahrensschritte auf meta-/para-Kresolgemische als Ausgangsprodukte befriedigt das seit langem bestehende Bedürfnis der wirtschaftlichen Erzeugung von 2,3,6-Trimethylphenol in bisher nicht erreichbarer Weise. Es ist dem Fachmann bekannt, daß sich die Fachwelt bisher vergeblich um eine Lösung dieser Aufgabe bemüht hat.

So geht eines der technisch wichtigsten Verfahren zur Herstellung von 2,3,6-Trimethylphenol, wie oben bereits ausgeführt, in eine völlig andere aufwendige Richtung.

Die langjährigen Untersuchungen der Anmelderin haben nunmehr zu einem Ergebnis geführt, das sicherlich eine sprunghafte Verbesserung des Standes der Technik darstellt und voraussichtlich auch die bisherigen Marktgegebenheiten einschneidend verändern wird.

In der Methylierungsstufe werden die Methylphenole mit Methanol und/oder Dimethylether einzeln oder im Gemisch verdampft und in den Reaktor eingeführt, in dem der Katalysator als Festbett angeordnet ist. Prinzipiell läßt sich die Umsetzung auch im Fließbett durchführen. Zusätzlich wird z. B. Wasserdampf in einer solchen Menge zugeführt, daß das molare Verhältnis Alkylphenole zu $H_2O$ etwa 1 zu 1-5 beträgt.

Der Reaktor wird bei 270-450 °C, bevorzugt bei 330-390 °C, betrieben. Die Umsetzung wird im allgemeinen bei Normaldruck oder bei geringem Überdruck durchgeführt, kann aber auch unter erhöhtem Druck durchgeführt werden bis ca. 100 bar. Bevorzugt wird die Umsetzung in der Gasphase bei Verweilzeiten von 0,01-10 Sekunden durchgeführt. Das molare Verhältnis von Alkylphenol zu Methanol und/oder Dimethylether liegt bei 1 : 0,1-10.

Nach der Methylierung kann (können) zunächst Dimethylphenol(e) und anschließend 2,4,6-Trimethylphenol abgetrennt werden oder beide können zusammen von 2,3,6-Trimethylphenol abgetrennt werden.

Die Isomerisierung/Transalkylierung wird bei 200-550 °C, vorzugsweise bei 250-450 °C, Drücken von 1-300 bar, vorzugsweise bei 5-180 bar, in vorzugsweise flüssiger Phase bei Verweilzeiten von 0,1-10 h, vorzugsweise von 0,5-7 Stdn. durchgeführt. Die Reaktion wird unter Inertgas, bevorzugt Stickstoff und/oder Wasserstoff durchgeführt, geeignet sind jedoch auch andere Inertgase wie z. B. $CO_2$, $CH_4$ oder Dampf.

Die umfangreichen Untersuchungen der Anmelderin haben ergeben, daß Eisenoxid(e) allein sowie Gemische von Eisenoxid(en) mit einer Vielzahl anderer Oxide hervorragende Ergebnisse liefern. Aus der Tabelle wird deutlich, daß sich die erfindungsgemäßen Katalysatoren dadurch auszeichnen, daß praktisch keine meta-substituierten Alkylphenole entstehen und sehr wenig Rückstand gebildet wird. Die Katalysatoren enthalten mindestens 60 Gew.% Eisenoxid(e). Bei kleinerem Eisenoxidanteil werden schlechtere Ergebnisse erhalten.

Der Katalysator kann in der Isomerisierung/Transalkylierung wie in der o-Methylierung als Fließbett vorliegen, wird jedoch bevorzugt als Festbett eingesetzt.

Anhand der folgenden Versuche wird die Erfindung im einzelnen erläutert.

Versuche

In eine kontinuierlich arbeitende Apparatur wurden pro Stunde 100 g eines technischen Gemisches von Alkylphenolen, gelöst in 145 g Methanol (molares Verhältnis 1 : 5) sowie 14 Gew.% Wasser bezogen auf das Gesamteinsatzprodukt eingesetzt. Das Alkylphenolgemisch bestand aus 55 Gew.% m-Kresol, 27 Gew.% p-Kresol, 7 Gew.% 2,5-, 8 Gew.% 2,4- und 3 Gew.% 2,6-Dimethylphenolen. In einem ersten mit Katalysator gefüllten Reaktionsraum erfolgte die o-Methylierung bei 5 bar, einer LHSV von 0,6 und einer Temperatur von 370 °C. Als Katalysator wurden Eisenoxid/Siliziumdioxid/Vanadiumoxid/Calciumoxid und in Parallelversuchen Eisenoxid/Siliziumdioxid/Chromoxid/Bariumoxid sowie Eisenoxid/Germaniumoxid/Chromoxid/Kaliumoxid eingesetzt. In allen Versuchen wurde o-Methylierung zu über 99 % erreicht. Der Anteil an Eisenoxid lag zwischen 90-100 Gew.% bezogen auf den Gesamtkatalysator.

Das Alkylierungsgemisch, das 78 g 2,3,6-Trimethylphenol, 42 g 2,4,6-Trimethylphenol und 3 g 2,6-Dimethylphenol enthielt, wurde nunmehr destillativ getrennt. Überschüssiges Methanol und Wasser wurden in üblicher Weise abgetrennt.

In einer ersten Destillationskolonne wurde über Kopf ein Gemisch von im wesentlichen 2,6-Dimethylphenol und 2,4,6-Trimethylphenol abdestilliert. Der Sumpf wurde in eine zweite Destillationskolonne eingesetzt, in der über Kopf 2,3,6-Trimethylphenol in über 99 %iger Reinheit abdestilliert wurde. Sehr geringe Mengen höher methylierter Phenole wurden aus dem Sumpf abgezogen und zusammen mit dem Kopfprodukt der ersten Kolonne in die Isomerisierungs-/Transalkylierungsstufe eingesetzt. Die Umsetzung erfolgte nach Zumischung von 43 g Phenol bei einer Temperatur von 400 °C, einer LHSV von 0,5 und einem Druck von 50-70 bar unter Inertgas. Als Katalysatoren wurden die oben angegebenen eingesetzt. Die Apparaturen wurden kontinuierlich 2 000 Stdn. betrieben, ohne daß nennenswerte Änderungen in Umsatz und Selektivität auftraten.

Die Isomerisierungs-/Transalkylierungsstufe wurde zusätzlich mit den in der Tabelle angegebenen Oxiden untersucht, wobei ebenfalls ausgezeichnete Ergebnisse bezüglich Selektivität und geringfügiger Rückstandsbildung erhalten wurden.

Der Produktgemisch wurde in üblicher Weise in die Einzelkomponenten getrennt.

Insgesamt läßt sich im kontinuierlichen Betrieb das Einsatzprodukt nahezu quantitativ zu o-Kresol, p-Kresol, 2,4- und 2,6-Dimethylphenol und 2,3,6-Trimethylphenol umsetzen.

In einer alternativen Fahrweise wurde in einer Vorkolonne Dimethylphenol über Kopf abdestilliert und in der oben genannten ersten Kolonne 2,4,6-Trimethylphenol abdestilliert, das zur Isomerisierung eingesetzt wurde. Es wurden im wesentlichen die gleichen Ergebnisse erhalten.

Analoge Ergebnisse werden mit reinen meta-/para-Kresolgemischen erhalten, wie z. B. mit einem Gemisch aus 60-70 Gew.% meta-Kresol und 30-40 Gew.% para-Kresol.

Erfindungsgemäß können selbstverständlich auch in Alkylierungsstufe und Isomerisierungs-/Transalkylierungsstufe verschiedene Katalysatoren verwendet werden. Die Versuche zeigen, daß sowohl andere Methylphenole enthaltende meta-/para-Kresolgemische als auch reine meta-/para-Kresolgemische mit sehr guten Ergebnissen bei dem erfindungsgemäßen Verfahren eingesetzt werden können.

(Siehe Tabelle Seite 5 f.)

Tabelle                                    Produktzusammensetzung in Gew.%

| Katalysator | Phenol | o-Kresol | p-Kresol | 2,4-DMP | 2,6-DMP | 2,4,6-TMP | Rückstand |
|---|---|---|---|---|---|---|---|
| $Fe_3O_4$ | 28,1 | 18,9 | 5,7 | 7,7 | 5,0 | 27,7 | 4,9 |
| $Fe_2O_3/Al_2O_3$ | 23,4 | 21,0 | 7,1 | 9,7 | 6,8 | 23,7 | 7,0 |
| $Fe_2O_3/Cr_2O_3/SiO_2/CaO$ | 25,6 | 19,3 | 7,5 | 14,9 | 8,3 | 21,5 | 2,3 |
| $Fe_2O_3MoO_2 K_2O$ | 24,3 | 19,5 | 6,6 | 14,5 | 9,9 | 24,0 | 1,2 |
| $Fe_2O_3/V_2O_5$ | 25,0 | 18,1 | 6,1 | 14,1 | 8,8 | 24,1 | 3,4 |
| $Fe_2O_3/SnO_2 Na_2O$ | 26,3 | 18,9 | 6,8 | 14,4 | 10,1 | 22,6 | 0,9 |
| $Fe_2O_3/NiO GeO_2/K_2O$ | 24,9 | 19,3 | 6,9 | 14,4 | 10,4 | 23,5 | 0,9 |
| $Fe_2O_3/Cr_2O_3 SiO_2/BaO$ | 25,2 | 19,1 | 7,8 | 17,0 | 8,6 | 21,4 | 0,4 |
| $Fe_2O_3/RuO_2$ | 25,2 | 18,8 | 6,5 | 13,7 | 8,8 | 24,2 | 2,3 |
| $Fe_2O_3/MgO SiO_2/BeO$ | 24,5 | 20,8 | 6,0 | 15,1 | 11,1 | 22,2 | 0,8 |
| $Fe_2O_3/MnO_2 K_2O$ | 24,9 | 19,7 | 6,1 | 14,5 | 10,6 | 22,4 | 1,8 |

DMP = Dimethylphenol
TMP = Trimethylphenol

0 154 830

**Patentansprüche**

1. Verfahren zur Umwandlung von meta-/para-Kresolgemischen und meta-/para-Kresolgemische enthaltenden Gemische, dadurch gekennzeichnet, daß das meta-/para-Kresolgemisch durch katalytische Umsetzung mit Methanol und/oder Dimethylether bei 270-450 °C bei einer Verweilzeit von 0,01-10 Sekunden und einem molaren Methylphenol zu Methanol- und/oder Dimethyletherverhältnis von 1 : 0,1-10 in ein Gemisch von 2,3,6-Trimethylphenol und 2,4,6-Trimethylphenol umgewandelt bzw. im wesentlichen umgewandelt wird, das 2,3,6-Trimethylphenol vom 2,4,6-Trimethylphenol abgetrennt bzw. im wesentlichen abgetrennt wird und das 2,4,6-Trimethylphenol oder ein im wesentlichen 2,4,6-Trimethylphenol enthaltendes Gemisch in Gegenwart von Phenol und/oder alkylierten Phenolen, in Gegenwart eines Katalysators, der aus Eisenoxid(en) besteht oder mindestens 60 Gew.% Eisenoxid(e) und wenigstens ein Oxid aus wenigstens einer der Gruppen

1. B, Al, Ce, Ga, In, Sc, Y
2. Si, Ge, Sn, Pb, Ti, Zr, Hf
3. Cr, V, Nb, Ta, Mo, W, Re, Co, Ni, Ru, Ir
4. Li, Na, K, Rb, Cs, Be, Mg, Ca, Sr, Ba, Mn, La, Cu, Zn, Cd

2. Verfahren nach Anspruch 1, dadurch gekennzeichnet, daß man die Isomerisierungs-/Transalkylierungsstufe in Wasserstoff- und/oder Stickstoff- und /oder Methan-Atmosphäre durchführt.

3. Verfahren nach den Anprüchen 1 und 2, dadurch gekennzeichnet, daß die o-Methylierung in der Gasphase und die Isomerisierung/Transalkylierung in der Flüssigphase durchgeführt werden.

**Claims**

1. Process for the conversion of meta/para-cresol mixtures, characterized in that the meta/para-cresol mixture is converted respectively essentially converted to a mixture of 2.3.6-trimethylphenol and 2.4.6-trimethylphenol by catalytic reaction with methanol and/or dimethylether at a temperature of 270-450 °C, at a residence time of 0,01-10 seconds and a molar ratio of methylphenol to methanol and/or dimethylether of 1 : 0,1-10, that 2.3.6-trimethylphenol is separated respectively essentially separated from 2.4.6-trimethylphenol and that 2.4.6-trimethylphenol resp. a mixture, which consists essentially of 2.4.6-trimethylphenol is converted in the presence of phenol and/or methylated phenols to a mixture of methylated phenols, which is essentially free of meta-substituted methylphenols, in the presence of a catalyst, which contains at least 60 weight-% of iron oxide(s), at a temperature of 200 to 550 °C, a pressure of 1-300 bar (at reaction temperature) and a residence time of 0,1-10 hours in inert atmosphere.

2. Process according to claim 1, characterized in that the isomerization/transalkylation is carried out in hydrogen and/or nitrogen and/or methane atmosphere.

3. Process according to claims 1 and 2, characterized in that the ortho-methylation is carried out in the gas phase and the isomerization/transalkylation in the liquid phase.

**Revendications**

1. Procédé pour la conversion de mélanges de méta-/para-crésols et de mélanges contenant des mélanges de méta-/para-crésols, caractérisé en ce que l'on convertit ou on convertit essentiellement un mélange de méta-/para-crésols en un mélange de 2,3,6-triméthylphénol et de 2,4,6-triméthylphénol par réaction catalytique avec le méthanol et/ou l'éther diméthylique à 270-450 °C avec une durée de passage de 0,01-10 s et un rapport molaire du méthylphénol au méthanol et/ou à l'éther diméthylique de 1 : 0,1-10, on sépare ou on sépare essentiellement le 2,3,6-triméthylphénol du 2,4,6-triméthylphénol et on transforme catalytiquement le 2,4,6-triméthylphénol ou un mélange contenant essentiellement du 2,4,6-triméthylphénol en présence de phénol et/ou de phénols alkylés en présence d'un catalyseur qui contient au moins 60 % en poids d'oxyde(s) de fer à des températures de 200-550 °C, sous des pressions de 1-300 bar (à la température de réaction) et avec des durées de passage de 0,1-10 h en un mélange de phénols méthylés qui est essentiellement exempt de méthylphénols méta-substitués.

2. Procédé selon la revendication 1, caractérisé en ce que l'on met en œuvre l'étape d'isomérisation/transalkylation en atmosphère d'hydrogène et/ou en atmosphère d'azote et/ou en atmosphère de méthane.

3. Procédé selon les revendications 1 et 2, caractérisé en ce que l'on effectue la o-méthylation en phase gazeuse et l'isomérisation/transalkylation en phase liquide.